# EUROPÄISCHE PATENTSCHRIFT

(11) **EP 0 722 455 B1**
(45) Veröffentlichungstag und Bekanntmachung des Hinweises auf die Patenterteilung: **19.08.1998**
(21) Anmeldenummer: 94929512.5
(22) Anmeldetag: 06.10.1994
(51) Int. Cl.: C07J 31/00

(54) **VERFAHREN ZUR HERSTELLUNG VON SCHWEFELSÄUREHALBESTERN**
PROCESS FOR PRODUCING SULFURIC ACID SEMI-ESTERS
PROCEDE DE PRODUCTION DE SEMI-ESTERS D'ACIDE SULPHURIQUE

(30) Priorität: 09.10.1993 DE 4334823
(43) Veröffentlichungstag der Anmeldung: 24.07.1996
(73) Patentinhaber: SCHERING AKTIENGESELLSCHAFT, 13342 Berlin (DE)
(72) Erfinder: JUNGHANS, Klaus, D-14163 Berlin (DE)
(86) Internationale Anmeldenummer: EP9403296
(87) Internationale Veröffentlichungsnummer: WO9510528

(56) Entgegenhaltungen:
- EP-A- 0 010 056
- EP-A- 0 117 570
- WO-A-93/17036

## Beschreibung

Die Erfindung betrifft ein Verfahren zur Herstellung von Schwefelsäurehalbestern der allgemeinen Formel I

RO - SO₂ - OH (I),

worin
- R: einen organischen Rest darstellt,
aus Hydroxyverbindungen der allgemeinen Formel II

R - OH (II),

worin
- R: die gleiche Bedeutung wie in Formel I besitzt.

Es sind bereits zahlreiche derartige Verfahren bekannt, die man als Sulfatierung bezeichnet (Römpp Chemie Lexikon 8te Aufl., 1979, p 4045 .- Franck'hsche Verlagshandlung, DE Stuttgart - und 9te Aufl. 1992, p 4376 - Georg Thieme Verlag, Stuttgart und New York). Bei diesem bekannten Verfahren werden als Sulfatierungsreagention Schwefelsäure, Oleum, Chlorsulfonsäure, Amidosulfonsäure oder Schwefeltrioxid verwendet. Das bei komplexen Hydroxyverbindungen wohl am häufigsten angewendete Sulfatierungsreagenz ist der Pyridin-Schwefeltrioxid-Komplex (Luis Fieser and Mary Fieser; Reagents for Organic Synthesis, John Wiley and Sons Inc. New York et al. Vol. 1, 1967, p 127, Vol. 2, 1969, p 393, Vol. 3, 1972, p 275, Vol. 4, 1974, p 473 und Vol. 9, 1981, p 396), mit dessen Hilfe unter anderem Schwefelsäurehalbester von aliphatischer Alkoholen, von Phenolen, von Hydroxysteroiden, von Kohlehydraten, oder von hydroxylgruppen haltigen Heterocyclen hergestellt wurden.

Dieses Reagenz hat aber nicht nur den Nachteil, daß bei seiner Anwendung oft erhebliche Mengen an unerwünschten Nebenprodukten gebildet werden. Gravierender noch ist der Umstand, daß die Herstellung des Pyrdin-Schwefeltrioxid-Komplexes selbst in einer ungewöhnlich stark exothermen Reaktion verläuft, was eine äußerst starke und somit aufwendige Kühlung erfordert, die insbesondere bei großtechnischen Reaktionen recht prolematisch und auch gefährlich ist. Hinzu kommt noch, daß eine umweltakzeptable Entsorgung der bei der Aufarbeitung der Reaktion anfallenden Schwefelsäure und Pyridin enthaltendes Abwässer nur unter erheblichem Aufwand möglich ist.

WO-A-93 170 36 offenbart außerden die Herstellung von Schwefelsäurehalbestern unter Verwendung von SO₃-Komplexen mit Trimethylamin. Zusätzlich-offenbart EP-A-10 056 die Verwendung von Sulfopropionsäureanhydrid bei der Herstellung derartiger Halbester.

Es wurde nun gefunden, daß es überraschenderweise möglich ist, die Schwefelsäurehalbester der allgemeinen Formel I aus den Hydroxyverbindungen der allgemeinen Formel II herzustellen, indem man diesen in einem inerten Lösungsmittel mit einem Dischwefelsäuresalz der allgemeinen Formel III

O(SO₂OX)₂ (III),

worin
- X: ein Alkalimetallatom symbolisiert
umsetzt.

Diese Reaktion dürfte sehr wahrscheinlich ähnlich universell anwendbar sein wie die Sulfatierung von Hydroxybindungen mit dem Pyridin-Schwefeltrioxid-Komplex, jedoch wurden im Rahmen der vorliegenden Untersuchungen bislang nur solche Hydroxyverbindungen der allgemeinen Formel II umgesetzt, in denen der Rest R einen Steroidrest darstellt.

Aus der Sicht der gewerblichen Verwertbarkeit gilt das Hauptinteresse der Herstellung solcher Schwefelsäurehalbester, die man als konjugierte Estrogene bezeichnet (konjugierte Estrogene sind beispielsweise in den Premarin® Tabletten der Firma Ayerst Labs., New York, enthalten; ausführliche Informationen zum Thema konjugierte Estrogene findet man beispielsweise in den Publikationen Pharmacopeial Forum, May, June 1991, p 1951-1962 und Journal of Chromatography 224, 1981, 355-370 sowie 234, 1982, 234-239).

Diese pharmakologisch wirksamen konjugierten Estrogene inclusive einiger Vorprodukte zu ihrer Herstellung sind gekennzeichnet durch die allgemeine Formel Ia 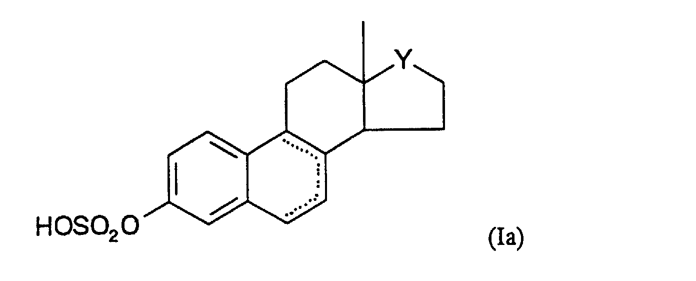 worin
die drei mit ....... gekennzeichneten Bindungen drei Einfachbindungen, zwei Einfachbindungen und eine Doppelbindungen oder zwei konjugierte Doppelbindungen symbolisieren und
Y eine Carbonylgruppe, eine Hydroxymethylengruppe, eine Acyloxymethylengruppe mit bis zu 8 Kohlenstoffatomen im Acylrest oder eine Benzyloxymethylengruppe darstellt.

Die konjugierten Estrogene der allgemeinen Formel Ia lassen sich mitttels des erfindungsgemäßen Verfahrens aus den entsprechenden Hydroxyverbindungen der allgemeinen Formel IIa 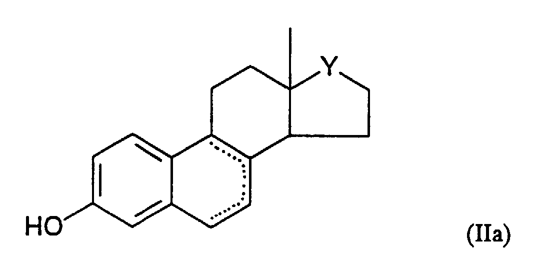 worin
die Bindungen ....... und Y die gleiche Bedeutung wie in Formel Ia besitzen, herstellen.

Lediglich bei Verbindungen der Formel IIa mit Y in der Bedeutung einer Hydroxymethylengruppe erhält man in der Regel nur recht geringe Ausbeuten an gewünschtem Verfahrensprodukt, da vorzugsweise diese Hydroxymethylengruppe in den Sulfatester überführt wird.

Zur Herstellung von konjugierten Estrogenen der allgemeinen Formel Ia mit einer freien 17α- oder 17β-Hydroxygruppen geht man zur Erzielung guter Ausbeuten meist zweckmäßigerweise von Hydroxyverbindungen der Formel IIa aus, deren 17 Hydroxygruppe verestert oder mit einer Benzylgruppe verethert ist, überführt diese in die entsprechenden konjugierten Estrogene der allgemeinen Formel Ia und entfernt die Schutzgruppen in bekannter Weise.

Das erfindungsgemäße Verfahren selbst ist in einfacher Weise durchführbar. Die Hydroxyverbindung der allgemeinen Formel I wird in einem inerten Lösungsmittel gelöst oder suspendiert, mit 1,05-1,5 mol Disulfat der Formel III versetzt und die Reaktionsmischung gegebenenfalls unter Rühren 1 Stunde bis 48.Stunden lang bei einer Temperatur von 0°C bis 60°C aufgewahrt.

Geeignete inerte Lösungsmittel sind beispielsweise chlorierte Kohlenwasserstoffe wie Dichlormethan oder Tetrachlorethan, Ether wie Diisopropylether, Dibutylether, 1,2-Dimethoxyethan, Tetrahydrofuran oder Dioxan, oder dipolare aprotische Lösungsmittel wie Dimethylformamid, N-Methylacetamid, Dimethylsulfoxid oder Hexamethylphosphorsäuretriamid.
Geeignete Disulfate sind insbesondere das Dinatrium- oder Dikaliumdisulfat.

Die Aufarbeitung der Reaktionsmischung kann in der gleichen Weise erfolgen, wie diejenige, die man bei der Sulfatierung von Hydroxyverbindungen mit dem Pyridin-Schwefeltrioxid-Komplex anwendet.
Die nachfolgenden Ausführungsbeispiele dienen zur näheren Erläuterung des erfindungsgemäßen Verfahrens.

### Beispiel 1

0,5 g 3-Hydroxy-estra-1,3,5(10)-trien-17-on und 0,5 g Natriumdisulfat werden mit 2 ml Dimethylfonnamid und 3 ml Tetrahydrofuran versetzt und 30 Stunden lang bei Raumtemperatur stehen gelassen. Dann setzt man der Reaktionsmischung 5 ml einer 1 n wässrigen Natriumcarbonatlösung zu, filtriert und engt das Filtrat im Vakuum ein. Der Rückstand wird in 50 ml Wasser aufgenommen, über Aktivkohle filtriert und das Filtrat gefriergetrocknet. Man erhält so 0,6 g des Natriumsalzes des 3-Hydroxy-estra-1,3 5(10)-trien-17-on-3-hemisulfat als farbloses Pulver vom Schmelzpunkt 270-277°C. [α]_{D} = +81,2° (Methanol).

### Beispiel 2

0,5 g 17α-Acetoxy-estra-1,3,5(10)-trien-3-ol in 2 ml Dimethyl-formamid werden 0,5 g Natriumdisulfat versetzt und 24 Stunden lang bei Raumtemperatur stehen gelassen. Dann versetzt man die Reaktionsmischung mit 10 ml einer 1n wässrigen Natriumcarbonat-Lösung und gibt sie nach 2 Stunden über eine Reversed-Phase-Chromatographie-Säule (J.Chem.Soc., Perkin Trans. I, 1987, 1339f). Man wäscht diese mit 20 ml destilliertem Wasser, eluiert mit Methanol, engt das Eluat im Vakuum ein und erhält so 0,5 g des Natriumsalzes des Estra-1,3,5(10)-trien-3,17β-diol-3-hydrogensulfat vom Schmelzpunkt 156-158°C (Zersetzung).
[α]_{D} = +40,3° (Methanol).

### Beispiel 3

0,5 g 17α-Acetoxy-estra-1,3,5(10),7-tetraen-3-ol werden mit 3 ml Dimethylformamid und 0,5 g Natriumdisulfat versetzt und 30 Stunden lang bei Raumtemperatur stehen gelassen. Man neutralisiert die Reaktionsmischung mit wässriger Natriumhydrogencarbonat-Lösung und engt sie im Vakuum ein. Der Rückstand wird mit 7 ml 1n wässriger Natronlauge versetzt, 1,5 Stunden lang bei Raumtemperatur stehen gelassen und mit 1n wässriger Schwefelsäure neutralisiert. Dann versetzt man die Reaktionsmischung mit 20 ml Isopropanol, filtriert und engt das Filtrat im Vakuum ein. Der Rückstand wird in 25 ml Wasser von 50°C gelöst, über Aktivkohle filtriert und das Filtrat im Vakuum eingeengt. Man erhält so 0,4 g des Natriumsalzes des Estra-1,3,5(10),7-tetraen-3,17α-diol-3-hemisulfats als beiges Pulver vom Schmelzpunkt 155-157°C (Zersetzung).
[α]_{D} = +155° (Methanol).

### Beispiel 4

0,5 g 17α-Acetoxy-estra-1,3,5(10),6,8-pentaen-3-ol werden mit 2 ml Dimethylformamid und 0,5 g Natriumdisulfat versetzt und 20 Stunden lang bei 20°C gerührt. Dann neutralisiert man die Reaktionsmischung mit 5 ml wässriger Natriumhydrogencarbonat-Lösung und engt sie im Vakuum zur Trockne ein. Der Rückstand wird in 15 ml in wässriger Natronlauge aufgenommen, 24 Stunden stehengelassen und mit Essigsäure neutralisiert. Die Reaktionsmischung wird auf eine Reversed-Phase-Chromatographie-Säule aufgebracht, mit Wasser gewaschen und mit Methanol eluiert. Das Eluat wird im Vakuum eingedampft und man erhält 0,4 g Estra-1,3,5(10),6.8-pentaen-3,17α-diol-3-hemisulfat-Natrium vom Schmelzpunkt 166°C.
[α]_{D} = -4,5° (Methanol).

### Beispiel 5

Unter den Bedingungen des Beispiels 2 werden 0,5 g 3-Hydroxy-estra-1,3,5(10),8-tetraen-17-on mit Natriumdisulfat umgesetzt, aufbereitet und man erhält 0,4 g 3-Hydroxy-estra-1,3,5(10),8-tetraen-17-en-3-hemisulfat-Natrium.

### Beispiel 6

Unter den Bedingungen des Beispiels 2 werden 0,5 g Estra-1,3,5(10),7-estratetraen-3.17β-diol mit Natrium disulfat umgesetzt, aufbereitet und man erhält 0,4 g Estra-1,3,5(10),7-tetraen-3,17β-diol-17-hemisulfat-Natrium.

## Patentansprüche

1. Verfahren zur Herstellung von Schwefelsäurehalbestern der allgemeinen Formel I
RO - SO₂ - OH (I),
worin
R einen organischen Rest darstellt,
aus Hydroxyverbindungen der allgemeinen Formel II
R - OH (II),
worin
R die gleiche Bedeutung wie in Formel I besitzt,
dadurch gekennzeichnet, daß man diese in einem inerten Lösungsmittel mit einem Dischwefelsäuresalz der allgemeinen Formel III
O(SO₂OX)₂ (III),
worin
X ein Alkalimetallatom symbolisiert
umsetzt.

2. Verfahren zur Herstellung von Schwefelsäurehalbestern der allgemeinen Formel I gemäß Patentanspruch 1, dadurch gekennzeichnet, daß R den Rest eines Hydroxysteroids symbolisiert.

3. Verfahren zur Herstellung von konjugierten Estrogenen der allgemeinen Formel Ia 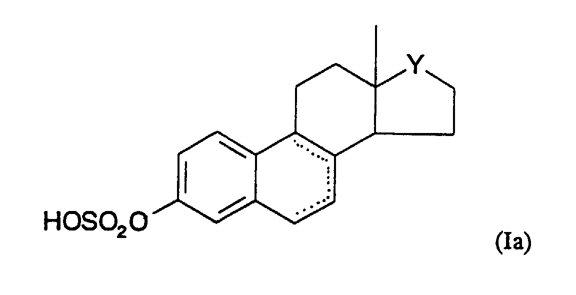 worin
die drei mit ....... gekennzeichneten Bindungen drei Einfachbindungen, zwei Einfachbindungen und eine Doppelbindungen oder konjugierte Doppelbindungen symbolisieren und
Y eine Carbonylgruppe, eine Hydroxymethylengruppe, eine Acyloxymethylengruppe mit bis zu 8 Kohlenstoffatomen im Acylrest oder eine Benzyloxymethylengruppe darstellt,
dadurch gekennzeichnet, daß man Hydroxysteroide der allgemeinen Formel IIa 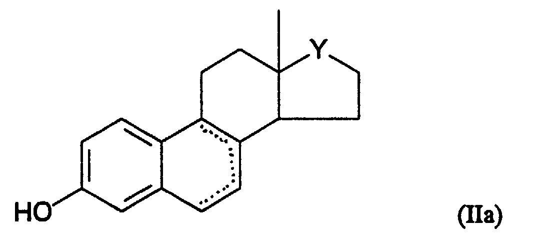 worin
die Bindungen ....... und Y die gleiche Bedeutung wie in Formel Ia besitzen, in einem inerten Lösungsmitel mit einem Dischwefelsäuresalz der allgemeinen Formel III
O(SO₂OX)₂ (III),
worin
X ein Alkalimetallatom symbolisiert
umsetzt.

## Claims

1. Process for the preparation of sulphuric acid semi-esters of the general formula I
RO - SO₂ - OH (I),
wherein
R represents an organic radical,
from hydroxy compounds of the general formula II
R - OH (II),
wherein
R has the same meaning as in formula I,
characterised in that
the latter are reacted in an inert solvent with a disulphuric acid salt of the general formula III
O(SO₂OX)₂ (III),
wherein
X represents an alkali metal atom.

2. Process for the preparation of sulphuric acid semi-esters of the general formula I according to patent claim 1, characterised in that R represents the radical of a hydroxy steroid.

3. Process for the preparation of conjugated oestrogens of the general formula la 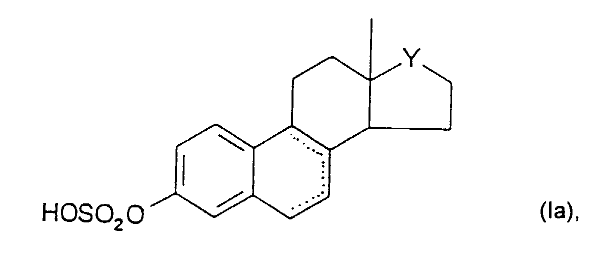 wherein
the three bonds indicated by ....... are three single bonds, two single bonds and one double bond, or conjugated double bonds and
Y represents a carbonyl group, a hydroxymethylene group, an acyloxymethylene group having up to 8 carbon atoms in the acyl radical or a benzyloxymethylene group,
characterised in that hydroxy steroids of the general formula IIa 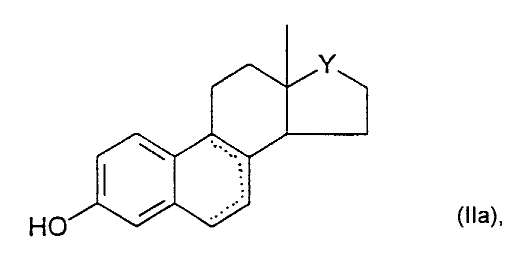 wherein
the bonds ....... and Y have the same meanings as in formula la,
are reacted in an inert solvent with a disulphuric acid salt of the general formula III
O(SO₂OX)₂ (III),
wherein
X represents an alkali metal atom.

## Revendications

1. Procédé de préparation de hémi-esters d'acide sulfurique répondant à la formule générale I
RO-SO₂-OH (I),
dans laquelle
R représente un radical organique,
à partir de composés hydroxy de la formule générale Il
R-OH (II),
dans laquelle R a la même signification que dans la formule I,
caractérisé en ce qu'on fait réagir ceux-ci dans un solvant inerte avec un sel d'acide disulfurique répondant à la formule générale III
O(SO₂OX)₂ (III),
dans laquelle X représente un atome de métal alcalin.

2. Procédé de préparation de hémi-esters d'acide sulfurique répondant à la formule générale I suivant la revendication 1, caractérisé en ce que R représente le radical d'un hydroxystéroïde.

3. Procédé de préparation d'oestrogènes conjugués de la formule générale la 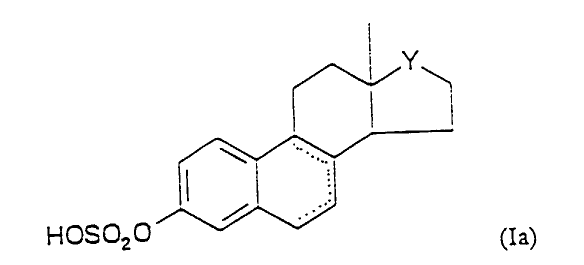 dans laquelle les liaisons représentées par ..... désignent trois liaisons simples, deux liaisons simples et une double liaison ou des doubles liaisons conjuguées, et
Y représente un groupe carbonyle, un groupe hydroxyméthylène, un groupe acyloxyméthylène comportant jusqu'à 8 atomes de carbone dans le radical acyle ou un groupe benzyloxyméthylène,
caractérisé en ce qu'on fait réagir des hydroxystéroïdes de la formule générale IIa 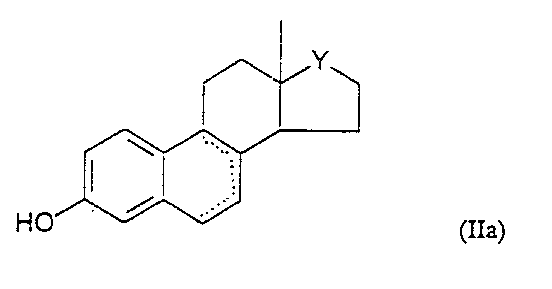 dans laquelle les liaisons ..... et Y ont la même signification que dans la formule la, dans un solvant inerte avec un sel d'acide disulfurique de la formule générale III
O(SO₂OX)₂ (III),
dans laquelle X représente un atome de métal alcalin.
